(19) Europäisches Patentamt
European Patent Office
Office européen des brevets

(11) Publication number: 0 384 917 B1

# (12) EUROPEAN PATENT SPECIFICATION

(45) Date of publication of patent specification: **13.01.93**

(51) Int. Cl.[5]: **C07C 225/18**, C07C 233/31, //C07D223/14

(21) Application number: **89103094.2**

(22) Date of filing: **22.02.89**

## (54) ( + )-Or ( + )-1-(2-N-substituted aminoethyl)-1-methyl-7-methoxy-1,2,3,4-tetrahydronaphthalene, and process for production thereof.

(43) Date of publication of application:
**05.09.90 Bulletin 90/36**

(45) Publication of the grant of the patent:
**13.01.93 Bulletin 93/02**

(84) Designated Contracting States:
**AT BE CH DE ES FR GB GR IT LI LU NL SE**

(56) References cited:

**CHEMICAL ABSTRACTS, vol. 104, no. 1, 6th January 1986, page 512, abstract no. 5644p, Columbus, Ohio, US; & JP-A-60 109 555**

(73) Proprietor: **Nihon Iyakuhin Kogyo Co., Ltd.**
**6-21, Sougawa 1-chome**
**Toyama City Toyama Prefecture(JP)**

(72) Inventor: **Nakamoto, Hiromasa**
**5-26, Marunouchi**
**Takaoka-shi Toyama-ken(JP)**
Inventor: **Ishizuka, Naoyasu**
**5-27, Ohmachi**
**Takaoka-shi Toyama-ken(JP)**
Inventor: **Takeda, Shigeki**
**3-12-14, Nishiyamadai**
**Osakasayama-shi Osaka-fu(JP)**
Inventor: **Yoshimura, Yoshifumi**
**105-2, Kitamitsubo Shinjyo-cho**
**Kitakatsuragi-gun Nara-ken(JP)**

(74) Representative: **Kraus, Walter, Dr. et al**
**Patentanwälte Kraus, Weisert & Partner**
**Thomas-Wimmer-Ring 15**
**W-8000 München 22(DE)**

EP 0 384 917 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to (±)- or (+)-1-(2-N-substituted aminoethyl)-1-methyl-7-methoxy-1,2,3,4-tetrahydronaphthalene represented by the following formula (A)

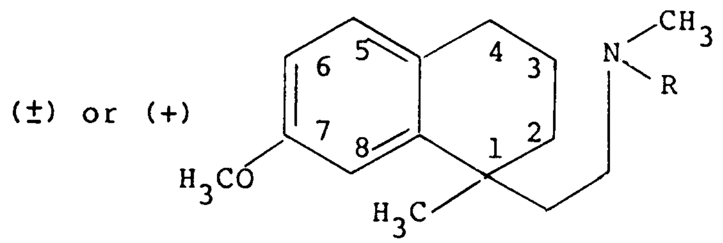

... (A)

wherein R represents H or $-COCH_3$, and when R represents $-COCH_3$, the compound is an optical (+) isomer,
which is useful, for example, as a synthesis intermediate for production of a pharmaceutical compound of formula (X) below known as "eptazocine hydrobromide". This invention also relates to a novel process for producing the compound of formula (A).

"Eptazocine hydrobromide" is another name for (-)-1,4-dimethyl-10-hydroxy-2,3,4,5,6,7-hexahydro-1,6-methano-1H-4-benzazonine hydrobromide represented by the following formula (X).

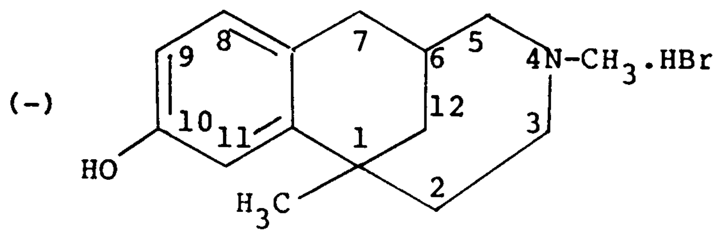

... (X)

It is a known pharmaceutical compound useful as a narcotic-antagonizing analgesic for relieving postoperative pains, pains from cancer, etc. The analgesic activity of the compound of formula (X) is described in detail, for example, in "Oyo Yakuri" (Applied Pharmacology), 19, (6), 973-982, 1980.

Japanese Laid-Open Patent Publication No. 109555/1985 (laid-open on June 15, 1985), of which inventorship partly overlaps that of the present application discloses 1-[2-(N-ethoxycarbonyl-N-methylamino)ethyl]-1-methyl-7-methoxy-1,2,3,4-tetrahydronaphthalene represented by the following formula (III)

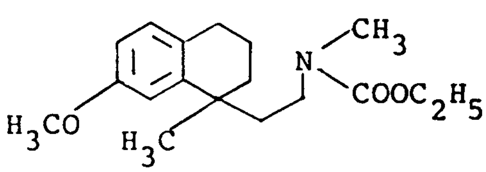

..... (III)

which can be used as a material for production of the compound of formula (A) provided by this invention, and a process for production of the compound of formula (III). This patent document discloses that the compound of formula (III) is useful as an intermediate for production of the compound of formula (X) through oxidation, hydrolysis, cyclization, reduction, optical resolution, hydrogenolysis and hydrobromide formation.

The present inventors made investigations in order to develop a process for producing the compound of formula (X) which is improved in operation and yield in industrial practice. Consequently, they succeeded in synthesizing the compound of formula (A) not described in the known literature from the known compound of formula (III), and discovered that the compound of formula (A) is a novel compound which is very useful as an intermediate for synthesis of the compound of formula (X).

In the invention of the above-cited Japanese Laid-Open Patent Publication No. 109555/1985, the optical

resolution is carried out in the latter half of the entire process for producing the compound of formula (X) from the compound of formula (III). In contrast, where optical resolution is necessary in the production of the compound of formula (X) from the compound of formula (A) in accordance with this invention, it can be carried out in the first half of its entire process in industrial-scale operation. Accordingly, the process for producing the compound of formula (X) from the compound of formula (A) provided by this invention is commercially very advantageous over the process disclosed in Japanese Laid-Open Patent Publication No. 109555/1985.

It is an object of this invention therefore to provide novel (±)- or (+)-1-(2-N-substituted aminoethyl)-1-methyl-7-methoxy-1,2,3,4-tetrahydronaphthalene useful as an intermediate for production of the compound of formula (X) which is a known drug, and a process for producing the compound of formula (A).

The above and other objects and advantages of this invention will become more apparent from the following description.

The (±)- or (+)-1-(2-N-substituted aminoethyl)1-methyl-7-methoxy-1,2,3,4-tetrahydronaphthalene of formula (A) provided by this invention is specifically (±)-1-(2-methyl aminoethyl)-1-methyl-7-methoxy-1,2,3,4-tetrahydronaphthalene represented by the following formula (II)

(±)
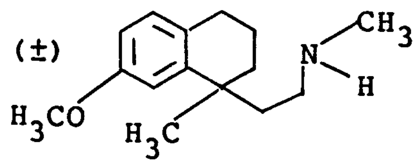
..... (II)

or (+)-1-(2-methylaminoethyl)-1-methyl-7-methoxy-1,2,3,4-tetrahydronaphthalene represented by the following formula (I)

(+)
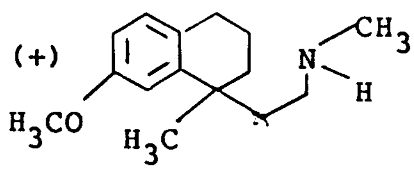
..... (I)

or (+)-1-[2-(N-acetyl-N-methylamino)ethyl]-1-methyl-7-methoxy-1,2,3,4-tetrahydronaphthalene represented by the formula (IV)

(+)
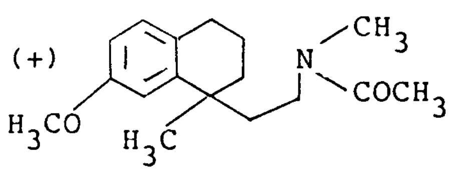
..... (IV).

The novel intermediate compound of formula (A) provided by this invention can be easily produced, for example, from 1-[2-(N-ethoxycarbonyl-N-methylamino)ethyl-1-methyl-7-methoxy-1,2,3,4-tetrahydronaphthalene of formula (III) which is easily available or can be easily produced.

According to this invention, there is also provided a process for producing (±)- or (+)-1-(2-N-substituted aminoethyl)-1-methyl-1,2,3,4-tetrahydronaphthalene represented by the following formula (A)

(±) or (+)
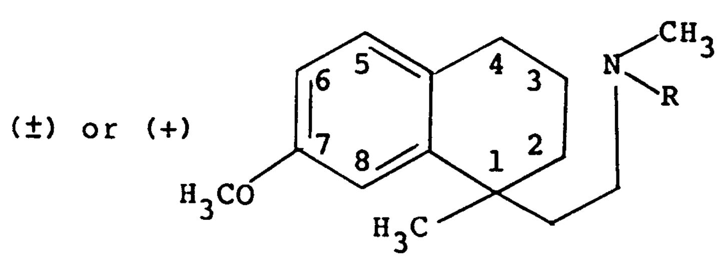
... (A)

wherein R represents H or $-COCH_3$, and when R represents $-COCH_3$, the compound is an optical (+) isomer,

which comprises

(a) reacting 1-[2-(N-ethoxycarbonyl-N-methylamino)ethyl]-1-methyl-7-methoxy-1,2,3,4-tetrahydronaphthalene represented by the following formula (III)

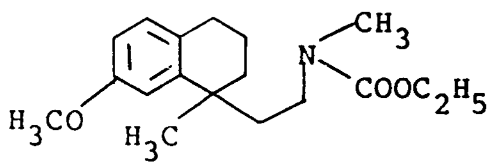

..... (III)

with an alkali hydroxide in a reaction solvent to form (±)-1-(2-methylaminoethyl)-1-methyl-7-methoxy-1,2,3,4-tetrahydronaphthalene represented by the following formula (II)

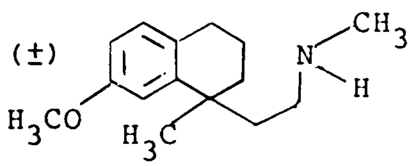

..... (II)

or (b) optically resolving (±)-1-(2-methylaminoethyl)-1-methyl-7-methoxy-1,2,3,4-tetrahydronaphthalene represented by the following formula (II)

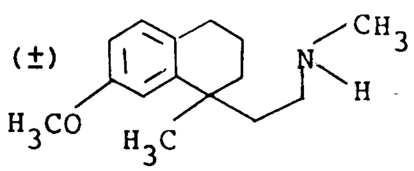

..... (II)

with optically active tartaric acid in a resolving solvent to form (+)-1-(2-methylaminoethyl)-1-methyl-7-methoxy-1,2,3,4-tetrahydronaphthalene represented by the following formula (I)

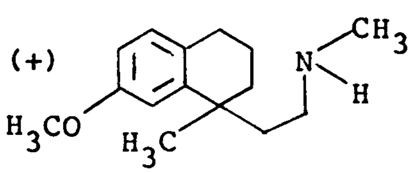

..... (I)

or (c) contacting (+)-1-(2-methylaminoethyl)-1-methyl-7-methoxy-1,2,3,4-tetrahydronaphthalene represented by the following formula (I)

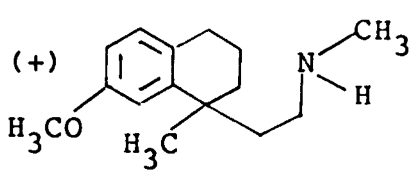

..... (I)

with an acetylating agent in a reaction solvent to form a compound of the following formula (IV)

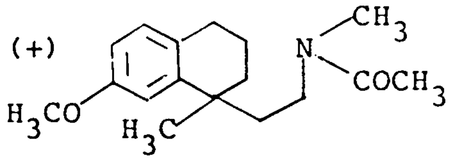

..... (IV).

The process for producing the compound of formula (A) in accordance with this invention by step (a), (b) or (c) is schematically shown below.

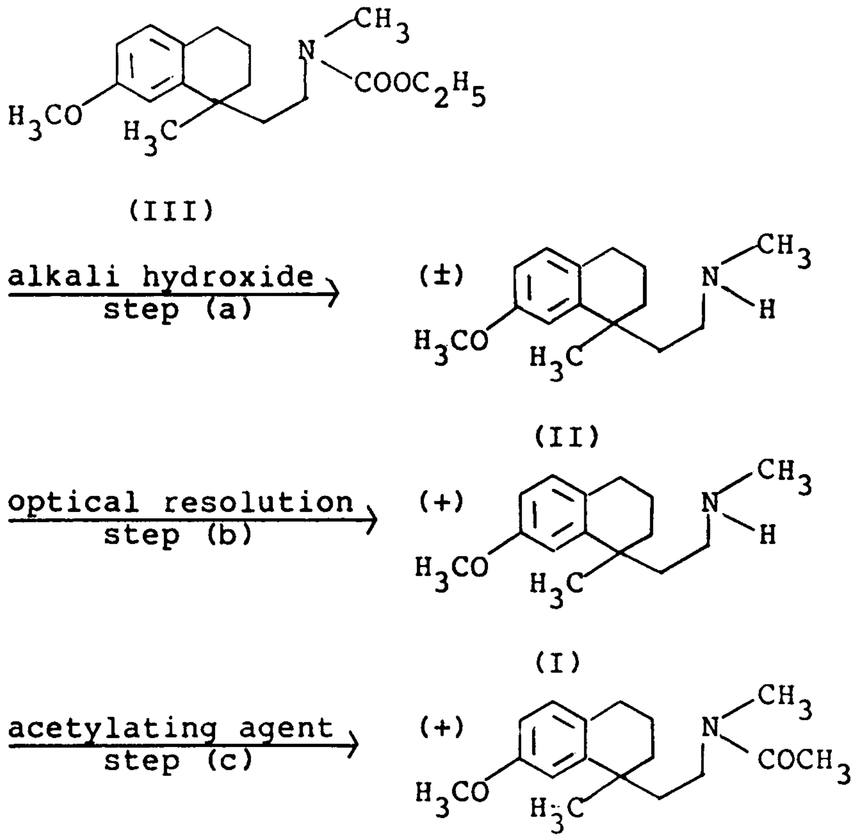


(III)

(II)

(I)

(IV)

The alkali hydroxide used in step (a) is, for example, potassium hydroxide or sodium hydroxide. The amount of the alkali metal hydroxide is preferably 3 to 6 moles per mole of the compound of formula (III). The reaction solvent used in this step may, for example, be a lower alcohol, preferably methanol or ethanol. The reaction in step (a) is preferably carried out, for example, by heating for 5 to 20 hours under reflux.

The optical resolution in step (b) can be carried out, for example, by dissolving the compound of formula (II) in (±) form in a suitable resolving solvent together with an optically active organic acid (resolving reagent) under heat to form the corresponding two diastereomers, cooling the solution overnight at about 0°C, precipitating one of the diastreomers as a sparingly-soluble crystalline salt by utilizing the differences between the solubilities of these diastereomers in the resolving solvent, and separating it from the other diastereomer left as a solution. One suitable optically active organic acid is, for example, D-(-)-tartaric acid or L-(+)-tartaric acid.

When D-(-)-tartaric acid is used, the diastereomer of the compound of formula (I) can be obtained as a sparingly-soluble crystalline salt. The amount of D-(-)-tartaric acid used is preferably 0.5 or 1 mole per mole of the compound of formula (II). Examples of the resolving solvent used are acetone, ethanol and a mixture of these in an arbitrary ratio. The amount of the resolving solvent may be properly selected, and is, for example, about 5 to about 20 parts by volume per part by volume of the compound of formula (II).

The crystalline salt of the diastereomer obtained as above is recrystallized from about 90% methanol, for example, to give pure crystalline salt of the (+)-form. The compound of formula (I) in (+)-form may be

obtained, for example, by adding an aqueous solution of an alkali hydroxide or ammonia to the crystalline salt of the (+)-form, and removing the resolving reagent using an extracting agent such as toluene or ethyl acetate.

The acetylating agent used in the acetylation in step (c) is a conventional acetylating agent such as acetic anhydride and acetyl chloride, the acetic anhydride being preferred. The amount of the acetylating agent used is preferably 1.5 to 4 moles per mole of the compound of formula (I). Glacial acetic acid and pyridine may be cited as examples of the reaction solvent used in step (c), and glacial acetic acid is preferred. The reaction may be carried out preferably by stirring at room temperature or under reflux for a period of, for example, 1 to 20 hours. Then, the unreacted acetylating agent and the reaction solvent are removed by, for example, distillation to obtain the compound of formula (IV).

An example of producing eptazocine hydrobromide of formula (X) using the compound of formula (II), (I) or (IV) will be described below.

This production may be shown by the following scheme.

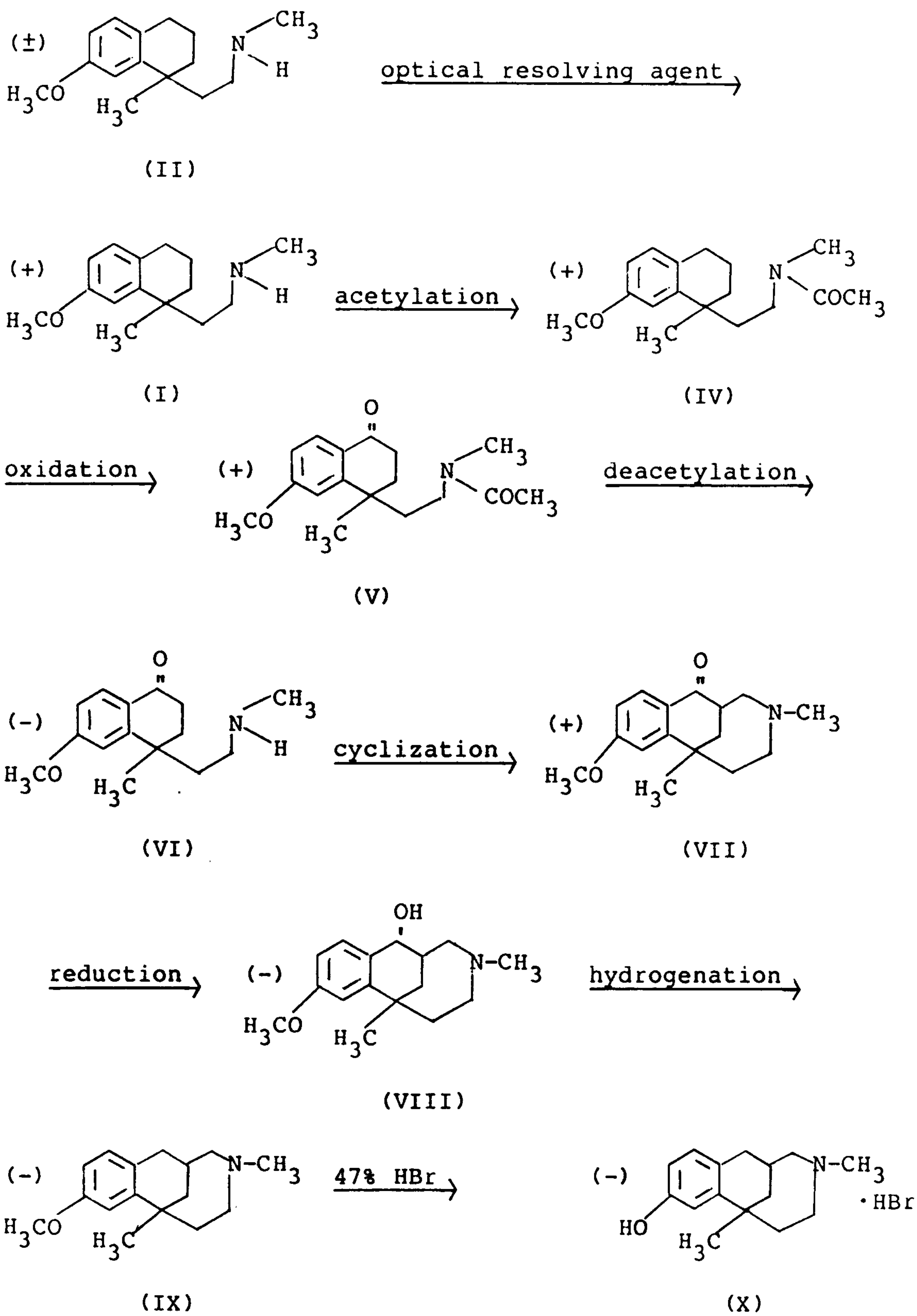


(II)

(I) (IV)

(V)

(VI) (VII)

(VIII)

(IX) (X)

The (+)-compound obtained by step (c) of the process of this invention may be converted to (+)-4-[2-(N-acetyl-N-methylamino)ethyl)]-4-methyl-6-methoxy-3,4-dihydro-1(2H)-naphthalenone represented by formula (V), not described in the prior literature, by subjecting it to oxidation reaction in accordance with a known technique. The reaction may be carried out by reacting it with chromium oxide in a reaction solvent such as acetic acid at a temperature of, for example, about 20 to 30 °Cfor a period of, for example, one

night.

The compound of formula (V) so obtained may be converted to known (-)-4-(2-methylaminoethyl)-4-methyl-6-methoxy-3,4-dihydo-1(2H)-naphthalenone of formula (VI) by refluxing it for about 5 hours together with an alkali such as potassium hydroxide in a reaction solvent such as methanol. The resulting (-)-compound of formula (VI) may be converted to known (+)-1,4-dimethyl-10-methoxy-2,3,4,5-tetrahydro-1,6-methano-1H-benzazonin-7(6H)-one of formula (VII) by cyclizing it by a known technique.

The cyclization reaction may be carried out, for example, by heating the compound of formula (VI) together with oxalic acid and paraformaldehyde in a reaction solvent such as methanol at a temperature of about 50 to 70 °C for a period of about 6 to 20 hours.

The resulting (+)-compound of formula (VII) may be converted to known (-)-1,4-dimethyl-7-hydroxy-10-methoxy-2,3,4,5,6,7-hexahydro-1,6-methano-1H-4-benzazonine of formula (VIII) by reducing its ketone group to the hydroxyl group.

This reducing reaction can be carried out by a known technique, for example by reacting it with sodium borohydride in a solvent such as methanol or ethanol at room temperature for 2 hours to one night.

The (-)-compound of formula (VIII) so obtained may be converted to known (-)-1,4-dimethyl-10-methoxy-2,3,4,5,6,7-hexahydro-1,6-methano-1H-4-benzazonine of formula (IX) by catalytic hydrogenation.

The hydrogenation reaction can be carried out, for example as described in Japanese Laid-Open Patent Publication No. 109555/1985, by contacting it with hydrogen gas and palladium-carbon in an acetic acid solvent in the presence of a mineral acid such as 70% perchloric acid or sulfuric acid at a temperature of, for example, about 20 to 30 °C, for a period of, for example, about 4 to 20 hours.

The compound of formula (IX) may also be produced by hydrogenolyzing the known compound of formula (VII).

The (-)-compound of formula (IX) obtained as above may be converted to (-)-1,4-dimethyl-10-hydroxy-2,3,4,5,6,7-hexahydro-1,6-methano-lH-4-benzazonine hydrobromide of formula (X), the known pharmaceutical compound also called eptazocine hydrobromide, by refluxing it with 47% hydrobromic acid for about 2 hours in accordance with a method described, for example, in Japanese Laid-Open Patent Publication No. 130843/1984.

The following examples illustrate the present invention in more detail.

EXAMPLE 1

Synthesis of (+)-1-(2-methylaminoethyl)-1-methyl-7-methoxy-1,2,3,4-tetrahydronaphthalene [compound of formula (I)]:-

Seventy grams of (±)-1-(2-methylaminoethyl)-1-methyl-7-methoxy-1,2,3,4-tetrahydronaphthalene and 22.5 g of D-(-)-tartaric acid were added to a mixture of 175 ml of acetone and 175 ml of ethanol, and the mixture was heated. The resulting solution was left to stand overnight in a refrigerator to precipitate crystals. The precipitated crystals were collected by filtration, and recrystallized from 90% methanol to give 28.5 g of (+)-1-(2-methylaminoethyl)-1-methyl-7-methoxy-1,2,3,4-tetrahydronaphthalene D-(-)-tartrate.

Melting point: 219.6 °C

$$[\alpha]_D^{24.5}:$$

+26.0 ° (c = 1.0, water)

To 28.5 g of the crystals were added 130 ml of water and 22 ml of 28% aqueous ammonia. The mixture was extracted with toluene. The resulting toluene solution was dried over anhydrous sodium sulfate, and the solvent was evaporated to give 21.6 g of the captioned compound as an oil.

Boiling point: 94 to 95 °C (0.07 torr)

$[\alpha]_D^{24}$ : +46.3 ° (c = 1.0, ethanol)

EXAMPLE 2

Synthesis of (±)-1-(2-methylaminoethyl)-1-methyl-7-methoxy-1,2,3,4-tetrahydronaphthalene [compound of formula (II)]:-

Potassium hydroxide (74.5 g) was added to a solution of 100 g of 1-[2-(N-ethoxycarbonyl-N-methylamino)ethyl]-1-methyl-7-methoxy-1,2,3,4-tetrahydronaphthalene in 137 ml of methanol, and the mixture was refluxed for 7 hours. Methanol was evaporated, and water was added to the residue. The mixture was extracted with toluene, and the toluene solution was extracted with dilute hydrochloric acid. The resulting acidic aqueous solution was neutralized with a dilute aqueous solution of sodium hydroxide. The liberated oil was extracted with toluene, and the resulting toluene solution was dried over anhydrous sodium sulfate. Toluene was then evaporated, and the residue was vacuum-distilled to give 54.3 g of the captioned compound as an oil.

Boiling point: 139 to 141 °C (2 torr)

EXAMPLE 3

Synthesis of (+)-1-[2-(N-acetyl-N-methylamino)-ethyl]-1-methyl-7-methoxy-1,2,3,4-tetrahydronaphthalene [compound of formula (IV)]:-

Acetic anhydride (65.2 g) and 14.4 ml of glacial acetic acid were added to 52.3 g of (+)-1-(2-methylaminoethyl)-1-methyl-7-methoxy-1,2,3,4-tetrahydronaphthalene, and the mixture was refluxed for 2 hours. Then, the unreacted acetic anhydride and glacial acetic acid were evaporated to give 61.7 g of the captioned compound as an oil.

Boiling point: 176 to 178 °C (1 torr)

$$[\alpha]_D^{23.5}:$$

+33.6° (c = 1.0, ethanol)

REFERENTIAL EXAMPLE 1

Synthesis of (-)-4-[2-(N-acetyl-N-methylamino)-ethyl]-4-methyl-6-methoxy-3,4-dihydro-1(2H)-naphthalenone [compound of formula (V)]:-

A solution of 40 g of chromium oxide in 40 ml of water was added dropwise at less than 25 °C to a solution of 61.9 g of (+)-1-[2-(N-acetyl-N-methylamino)-ethyl]-1-methyl-7-methoxy-1,2,3,4-tetrahydronaphthalene in 140 ml of acetic acid, and the mixture was stirred overnight at the above temperature. Then, 140 ml of methanol was added, and the mixture was further stirred for 30 minutes. The solvent was evaporated. The residue was mixed with water and then extracted with toluene. The extract was washed with a saturated aqueous solution of sodium hydrogen carbonate, and dried over anhydrous magnesium sulfate. The solvent was evaporated to give 61.7 g of the captioned compound as an oil.

$[\alpha]_D^{28}$ : -15.7 ° (c = 1.0, ethanol)

REFERENTIAL EXAMPLE 2

Synthesis of (-)-4-(2-methylaminoethyl)-4-methyl-6-methoxy-3,4-dihydro-1(2H)-naphthalenone compound of formula (VI)]:-

Potassium hydroxide (47.8 g) was added to a solution of 61.7 g of (-)-4-[2-(N-acetyl-N-methylamino)-ethyl]-4-methyl-6-methoxy-3,4-dihydro-1(2H)-naphthalenone in 72 ml of methanol, and the mixture was heated under reflux for 5 hours. After the reaction, water was added, and the reaction mixture was extracted with toluene. The toluene solution was extracted with dilute hydrochloric acid. The resulting aqueous solution was neutralized with a dilute aqueous solution of sodium hydroxide. The neutralized solution was extracted with toluene, and the toluene solution was dried over potassium carbonate. The solvent was evaporated to give 47.5 g of the captioned compound as an oil.

$[\alpha]_D^{21}$ : -16.4 ° (c = 1.0, ethanol)

REFERENTIAL EXAMPLE 3

Synthesis of (+)-1,4-dimethyl-10-methoxy-2,3,4,5-tetrahydro-1,6-methano-1H-4-benzazonin-7(6H)-one [compound of formula (VII)]:-

A mixture of 47.5 g of (-)-4-(2-methylaminoethyl)-4-methyl-6-methoxy-3,4-dihydro-1(2H)-naphthalenone, 18.2 g of 95% paraformaldehyde, 24.2 g of oxalic acid and 566 ml of methanol was stirred at 50 to 55 °C for 14 hours. The solvent was then evaporated. The residue was dissolved in 300 ml of water and neutralized with an aqueous solution of potassium hydroxide. The neutralized solution was extracted with toluene, and the toluene solution was dried over anhydrous sodium sulfate. The solvent was evaporated to give 49.8 g of the captioned compound as an oil.

$[\alpha]_D^{20}$ : +5.1 ° (c = 1.15, ethanol)

REFERENTIAL EXAMPLE 4

Synthesis of (-)-1,4-dimethyl-7-hydroxy-10-methoxy-2,3,4,5,6,7-hexahydro-1,6-methano-1H-4-benzazonine [compound of formula (VIII)]:-

Sodium borohydride (4.4 g) was added to a solution of 51.5 g of (+)-1,4-dimethyl-10-methoxy-2,3,4,5-tetrahydro-1,6-methano-1H-4-benzazonin-7(6H)-one in 235 ml of ethanol, and the mixture was stirred overnight at room temperature. Ethanol was then evaporated, and 200 ml of water was added to the residue. The liberated oil was extracted with dichloromethane, and further with dilute hydrochloric acid. The extracted aqueous solution was neutralized with a dilute aqueous solution of sodium hydroxide. The liberated oil was extracted with chloroform. Chloroform was evaporated. The residue was dissolved in benzene and the solution was left to stand overnight in a refrigerator. The precipitated crystals were collected by filtration to give 26.9 g of the captioned compound.

Melting point: 167.9 °

$$[\alpha]_D^{26.5}:$$

-42.9 ° (c = 1.0, ethanol)

REFERENTIAL EXAMPLE 5

Synthesis of (-)-1,4-dimethyl-10-methoxy-2,3,4,5,6,7-hexahydro-1,6-methano-1H-4-benzazonine [compound of formula (IX)]:-

79.5 g of (-)-1,4-dimethyl-7-hydroxy-10-methoxy-2,3,4,5,6,7-hexahydro-1,6-methano-1H-4-benzazonine was dissolved in 400 ml of glacial acetic acid. A suspension of 47.7 g of 5% palladium-carbon in 400 ml of glacial acetic acid was added, and further 40 ml of 62.5% sulfuric acid was added. The mixture was stirred vigorously for 6 hours at room temperature while hydrogen gas was introduced into it. The palladium-carbon was removed by filtration, and the filtrate was distilled under reduced pressure to remove acetic acid. The residue was dissolved in water and neutralized with a dilute aqueous solution of sodium hydroxide. The liberated oil was extracted with toluene. The toluene solution was dried over potassium carbonate, and toluene was evaporated. The residue was vacuum-distilled to give 66.6 g of the captioned compound as an oil.

Boiling point: 154 to 156 °C (1 torr)

$[\alpha]_D^{23}$ : -16.0 ° (c = 1.0, ethanol)

REFERENTIAL EXAMPLE 6

Synthesis of (-)-1,4-dimethyl-10-hydroxy-2,3,4,5,6,7-hexahydro-1,6-methano-1H-4-benzazonine hydro-bromide [compound of formula (X)]:-

A solution of 200 g of (-)-1,4-dimethyl-10-methoxy-2,3,4,5,6,7-hexahydro-1,6-methano-1H-4-benzazonine in 800 ml of 47% hydrobromic acid was slowly refluxed for 2 hours. After the reaction, water and other volatile materials were evaporated under reduced pressure. The resulting crude crystals were recrystallized from ethanol to obtain 188.31 g of the captioned compound as crystals.

Melting point: 259.3 °C

$[\alpha]_D^{25}$ : -15.4 ° (c = 4.87, water)

**Claims**

**Claims for the following Contracting States : AT, BE, CH, DE, FR, GB, GR, IT, LU, NL, SE**

1. (±)- or (+)-1-(2-N-substituted aminoethyl)-1-methyl-7-methoxy-1,2,3,4-tetrahydronaphthalene represented by the following formula (A)

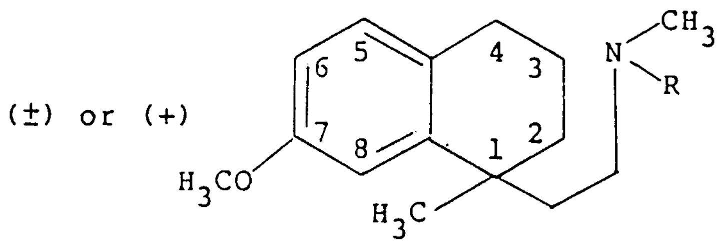

... (A)

wherein R represents H or $-COCH_3$, and when R represents $-COCH_3$, the compound is an optical isomer (+).

2. The compound of claim 1 which is (±)-1-(2-methylaminoethyl)-1-methyl-7-methoxy-1,2,3,4-tetrahydronaphthalene represented by the following formula (II):

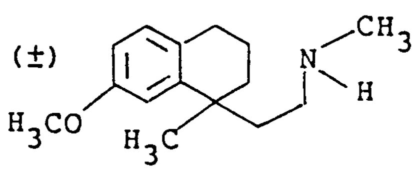

..... (II)

3. The compound of claim 1 which is (+)-1-(2-methylaminoethyl)-1-methyl-7-methoxy-1,2,3,4-tetrahydronaphthalene represented by the following formula (I):

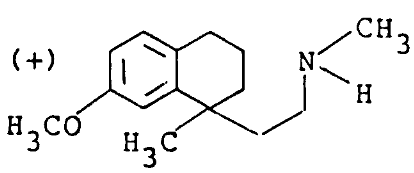

..... (I)

4. The compound of claim 1 which is (+)-1-[2-(N-acetyl-N-methylamino)ethyl]-1-methyl-7-methoxy-1,2,3,4-tetrahydronaphthalene represented by the following formula (IV):

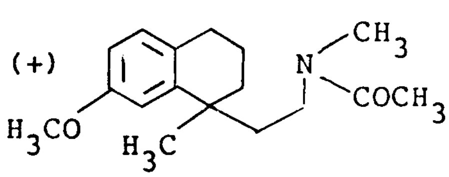

..... (IV)

5. A process for producing (±)- or (+)-1-(2-N-substituted aminoethyl)-1-methyl-1,2,3,4-tetrahydronaphthalene represented by the following formula (A)

(±) or (+) 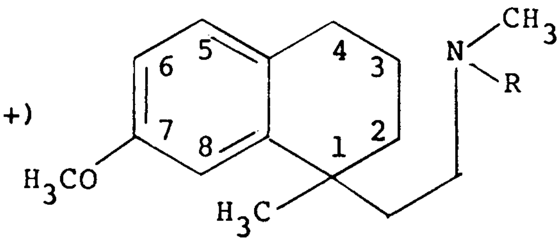 ... (A)

wherein R represents H or $-COCH_3$, and when R represents $-COCH_3$, the compound is an optical (+) isomer,
which comprises

(a) reacting 1-[2-(N-ethoxycarbonyl-N-methylamino)ethyl]-1-methyl-7-methoxy-1,2,3,4-tetrahydronaphthalene represented by the following formula (III)

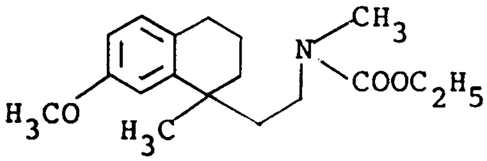 ..... (III)

with an alkali hydroxide in a reaction solvent to form (±)-1-(2-methylaminoethyl)-1-methyl-7-methoxy-1,2,3,4-tetrahydronaphthalene represented by the following formula (II)

(±) 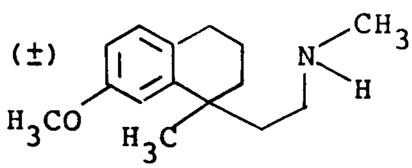 ..... (II)

or (b) optically resolving (±)-1-(2-methylaminoethyl)-1-methyl-7-methoxy-1,2,3,4-tetrahydronaphthalene represented by the following formula (II)

(±) 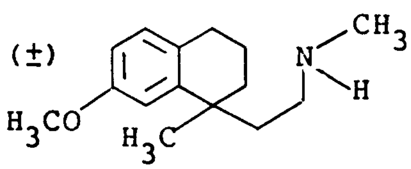 ..... (II)

with optically active tartaric acid in a resolving solvent to form (+)-1-(2-methylaminoethyl)-1-methyl-7-methoxy-1,2,3,4-tetrahydronaphthalene represented by the following formula (I)

(+) 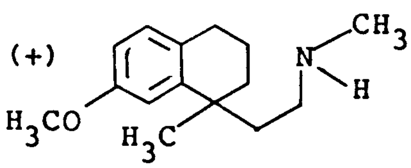 ..... (I)

or (c) contacting (+)-1-(2-methylaminoethyl)-1-methyl-7-methoxy-1,2,3,4-tetrahydronaphthalene represented by the following formula (I)

(+)

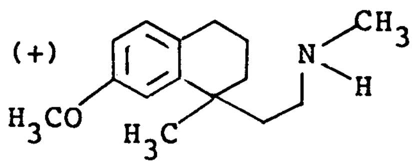

..... (I)

with an acetylating agent in a reaction solvent to form a compound of the following formula (IV)

(+)

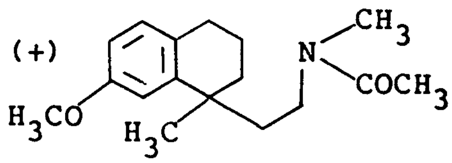

..... (IV).

**Claims for the following Contracting State : ES**

1. A process for producing (±)- or (+)-1-(2-N-substituted aminoethyl)-1-methyl-1,2,3,4-tetrahydronaphthalene represented by the following formula (A)

(±) or (+)

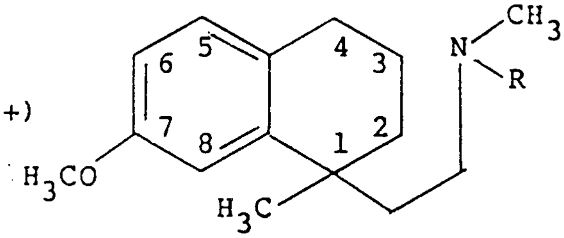

... (A)

wherein R represents H or $-COCH_3$, and when R represents $-COCH_3$, the compound is an optical (+) isomer,
which comprises
(a) reacting 1-[2-(N-ethoxycarbonyl-N-methylamino)ethyl]-1-methyl-7-methoxy-1,2,3,4-tetrahydronaphthalene represented by the following formula (III)

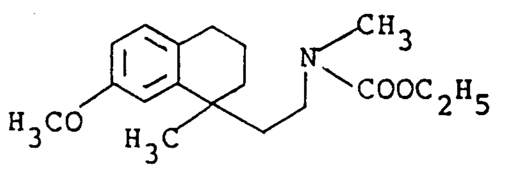

..... (III)

with an alkali hydroxide in a reaction solvent to form (±)-1-(2-methylaminoethyl)-1-methyl-7-methoxy-1,2,3,4-tetrahydronaphthalene represented by the following formula (II)

(±)

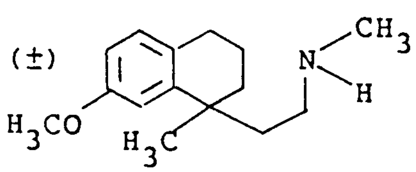

..... (II)

or (b) optically resolving (±)-1-(2-methylaminoethyl)-1-methyl-7-methoxy-1,2,3,4-tetrahydronaphthalene represented by the following formula (II)

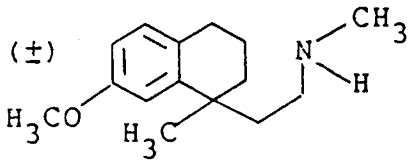

..... (II)

with optically active tartaric acid in a resolving solvent to form (+)-1-(2-methylaminoethyl)-1-methyl-7-methoxy-1,2,3,4-tetrahydronaphthalene represented by the following formula (I)

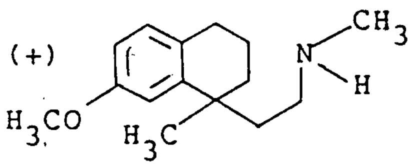

..... (I)

or (c) contacting (+)-1-(2-methylaminoethyl)-1-methyl-7-methoxy-1,2,3,4-tetrahydronaphthalene represented by the following formula (I)

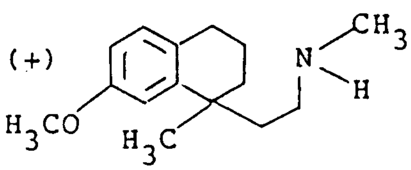

..... (I)

with an acetylating agent in a reaction solvent to form a compound of the following formula (IV)

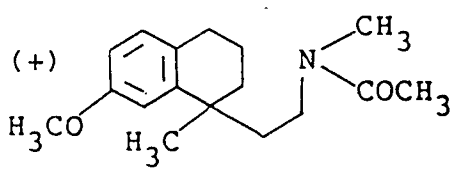

..... (IV).

2. The process of claim 1 for the production of (±)-1-(2-methylaminoethyl)-1-methyl-7-methoxy-1,2,3,4-tetrahydronaphthalene represented by the following formula (II):

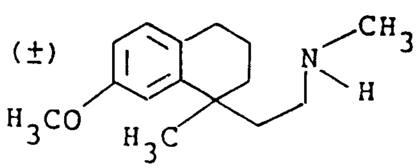

..... (II)

3. The process of claim 1 for the production of (+)-1-(2-methylaminoethyl)-1-methyl-7-methoxy-1,2,3,4-tetrahydronaphthalene represented by the following formula (I):

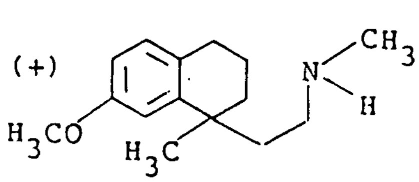

..... (I)

4. The process of claim 1 for the production of (+)-1-[2-(N-acetyl-N-methylamino)ethyl]-1-methyl-7-methoxy-1,2,3,4-tetrahydronaphthalene represented by the following formula (IV):

(+) 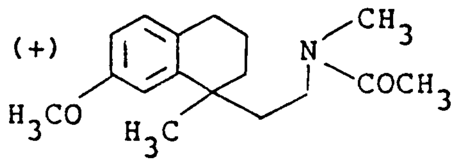 ..... (IV)

**Patentansprüche**
**Patentansprüche für folgende Vertragsstaaten : AT, BE, CH, DE, FR, GB, GR, IT, LI, LU, NL, SE**

1. (±)- oder (+)-1-(2-N-substituiertes Aminoethyl)-1-methyl-7-methoxy-1,2,3,4-tetrahydronaphthalin der folgenden Formel (A):

(±) oder (+) 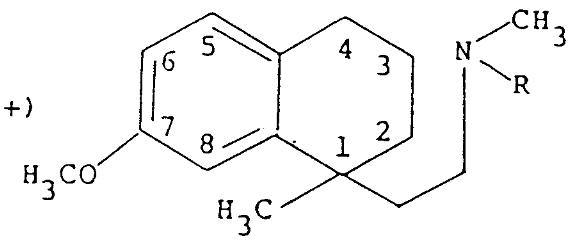 ... (A)

worin A H oder $-COCH_3$ bedeutet, und wenn A $-COCH_3$ bedeutet, die Verbindung das optische Isomere (+) ist.

2. Verbindung nach Anspruch 1, nämlich (±)-1-(2-Methylaminoethyl)-1-methyl-7-methoxy-1,2,3,4-tetrahydronaphthalin der folgenden Formel (II):

(±) 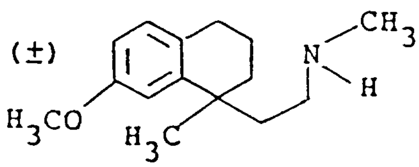 ..... (II)

3. Verbindung nach Anspruch 1, nämlich (+)-1-(2-Methylaminoethyl)-1-methyl-7-methoxy-1,2,3,4-tetrahydronaphthalin der folgenden Formel (I):

(+) 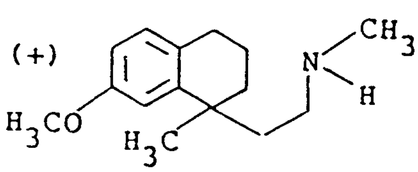 ..... (I)

4. Verbindung nach Anspruch 1, nämlich (+)-1-[2-(N-Acetyl-N-methylamino)-ethyl]-1-methyl-7-methoxy-1,2,3,4-tetrahydronaphthalin der folgenden Formel (IV):

(+) 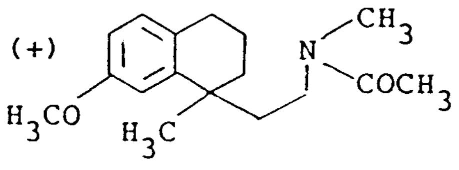 ..... (IV)

**5.** Verfahren zur Herstellung von (±)- oder (+)-1-(2-N-substituiertem Aminoethyl)-1-methyl-1,2,3,4-tetrahydronaphthalin der folgenden Formel (A):

(±) oder (+) 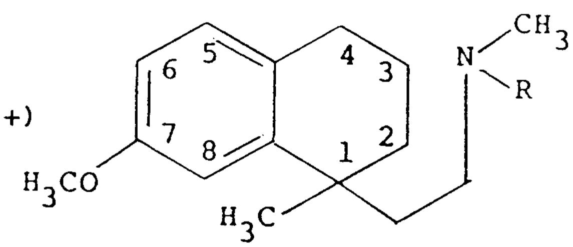 ... (A)

worin R H oder $-COCH_3$ bedeutet, und wenn A $-COCH_3$ bedeutet, die Verbindung das optische Isomere (+) ist,

dadurch **gekennzeichnet,** daß

(a) 1-[2-(N-Ethoxycarbonyl-N-methylamino)-ethyl]-1-methyl-7-methoxy-1,2,3,4-tetrahydronaphthalin der folgenden Formel (III):

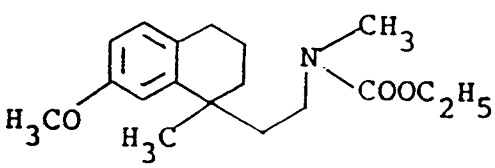 ..... (III)

mit einem Alkalihydroxid in einem Reaktions-Lösungsmittel unter Bildung von (±)-1-(2-Methylaminoethyl)-1-methyl-7-methoxy-1,2,3,4-tetrahydronaphthalin der folgenden Formel (II):

(±) 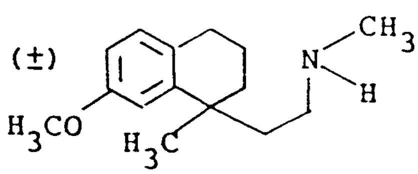 ..... (II)

umgesetzt wird; oder

(b) (±)-1-(2-Methylaminoethyl)-1-methyl-7-methoxy-1,2,3,4-tetrahydronaphthalin der folgenden Formel (II):

(±) 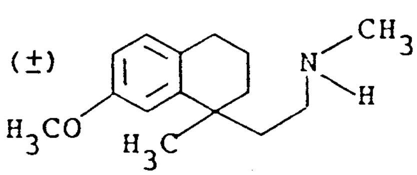 ..... (II)

mit einer optisch aktiven Weinsäure in einem Aufspaltungs-Lösungsmittel unter Bildung von (+)-1-(2-Methylaminoethyl)-1-methyl-7-methoxy-1,2,3,4-tetrahydronaphthalin der folgenden Formel (I):

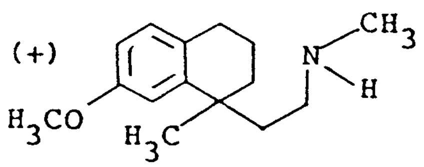

..... (I)

optisch aufgespalten wird; oder

(c) (+)-1-(2-Methylaminoethyl)-1-methyl-7-methoxy-1,2,3,4-tetrahydronaphthalin der folgenden Formel (I):

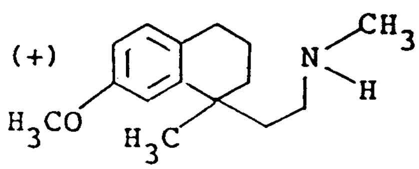

..... (I)

mit einem Acetylierungsmittel in einem Reaktions-Lösungsmittel unter Bildung einer Verbindung der folgenden Formel (IV):

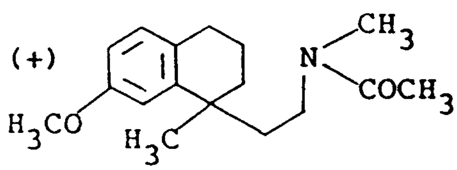

..... (IV)

behandelt wird.

**Patentansprüch für folgenden Vertragsstaat : ES**

**1.** Verfahren zur Herstellung von (±)- oder (+)-1-(2-N-substituiertem Aminoethyl)-1-methyl-1,2,3,4-tetrahydronaphthalin der folgenden Formel (A):

(±) oder (+)

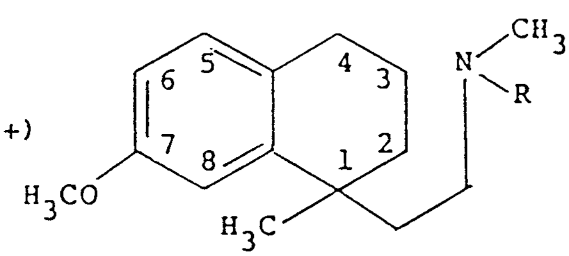

... (A)

worin R H oder $-COCH_3$ bedeutet, und wenn A $-COCH_3$ bedeutet, die Verbindung das optische Isomere (+) ist,

dadurch **gekennzeichnet,** daß

(a) 1-[2-(H-Ethoxycarbonyl-N-methylamino)-ethyl]-1-methyl-7-methoxy-1,2,3,4-tetrahydronaphthalin der folgenden Formel (III):

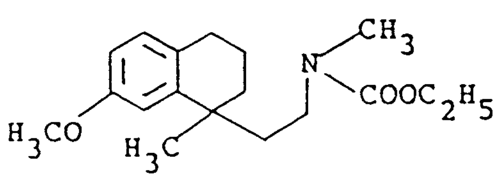

..... (III)

mit einem Alkalihydroxid in einem Reaktions-Lösungsmittel unter Bildung von (±)-1-(2-Methylaminoethyl)-1-methyl-7-methoxy-1,2,3,4-tetrahydronaphthalin der folgenden Formel (II):

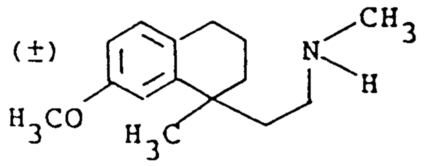

..... (II)

umgesetzt wird; oder
(b) (±)-1-(2-Methylaminoethyl)-1-methyl-7-methoxy-1,2,3,4-tetrahydronaphthalin der folgenden Formel (II):

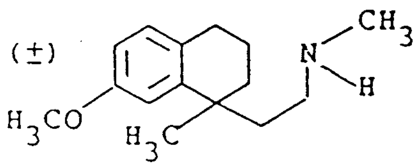

..... (II)

mit einer optisch aktiven Weinsäure in einem Aufspaltungs-Lösungsmittel unter Bildung von (+)-1-(2-Methylaminoethyl)-1-methyl-7-methoxy-1,2,3,4-tetrahydronaphthalin der folgenden Formel (I):

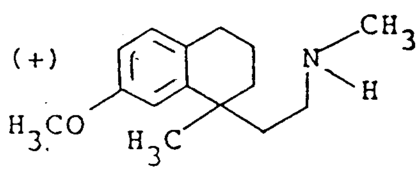

..... (I)

optisch aufgespalten wird; oder
(c) (+)-1-(2-Methylaminoethyl)-1-methyl-7-methoxy-1,2,3,4-tetrahydronaphthalin der folgenden Formel (I):

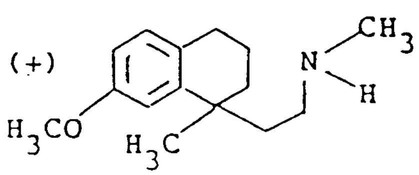

..... (I)

mit einem Acetylierungsmittel in einem Reaktions-Lösungsmittel unter Bildung einer Verbindung der folgenden Formel (IV):

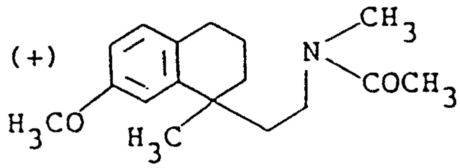

..... (IV).

behandelt wird.

2. Verfahren nach Anspruch 1, dadurch **gekennzeichnet,** daß (±)-1-(2-Methylaminoethyl)-1-methyl-7-methoxy-1,2,3,4-tetrahydronaphthalin der folgenden Formel (II):

(±) 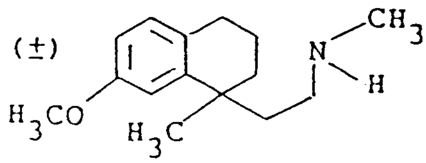 ..... (II)

hergestellt wird.

**3.** Verfahren nach Anspruch 1, dadurch **gekennzeichnet,** daß (+)-1-(2-Methylaminoethyl)-1-methyl-7-methoxy-1,2,3,4-tetrahydronaphthalin der folgenden Formel (I):

(+) 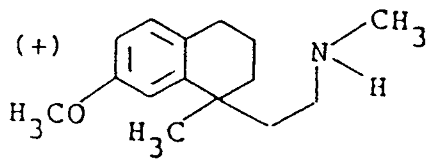 ..... (I)

hergestellt wird.

**4.** Verfahren nach Anspruch 1, dadurch **gekennzeichnet,** daß (+)-1-[2-(N-Acetyl-N-methylamino)-ethyl]-1-methyl-7-methoxy-1,2,3,4-tetrahydronaphthalin der folgenden Formel (IV):

(+) 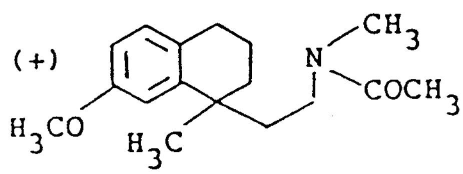 ..... (IV)

hergestellt wird.

## Revendications

**Revendications pour les Etats contractants suivants : AT, BE, CH, DE, FR, GB, GR, IT, LU, NL, SE, LI**

**1.** (±)- ou (+)-1-[2-(amino N-substitué)éthyl]-1-méthyl-7-méthoxy-1,2,3,4-tétrahydronaphtalène représenté par la formule (A) suivante

(±) ou (+) 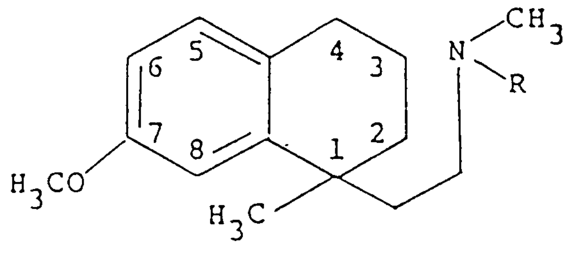 ... (A)

dans laquelle R représente H ou $-COCH_3$ et, si R représente $-COCH_3$, le composé est un isomère optique (+).

**2.** Composé selon la revendication 1, qui est le (±)-1-(2-méthylaminoéthyl)-1-méthyl-7-méthoxy-1,2,3,4-tétrahydronaphtalène représenté par la formule (II) suivante :

(±) 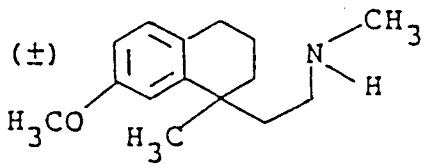 ..... (II)

3. Composé selon la revendication 1, qui est le (+)-1-(2-méthylaminoéthyl)-1-méthyl-7-méthoxy-1,2,3,4-tétrahydronaphtalène représenté par la formule (I) suivante :

(+) 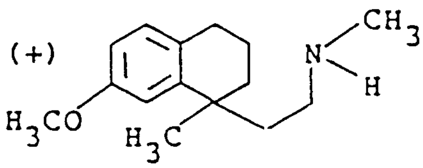 ..... (I)

4. Composé selon la revendication 1, qui est le (+)-1-[2-(N-acétyl-N-méthylamino)éthyl]-1-méthyl-7-méthoxy-1,2,3,4-tétrahydronaphtalène représenté par la formule (IV) suivante :

(+) 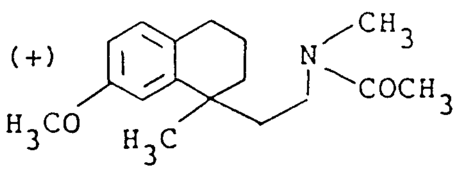 ..... (IV)

5. Procédé de production de (±)- ou (+)-1-[2-(amino N-substitué)éthyl]-1-méthyl-1,2,3,4-tétrahydronaphtalène représenté par la formule (A) suivante

(±) ou (+) 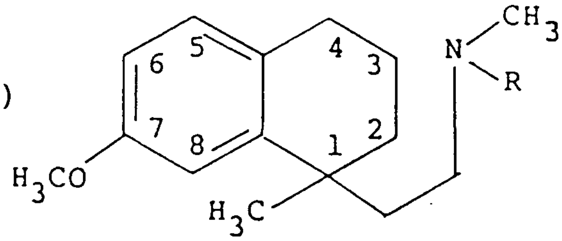 ..... (A)

dans laquelle R représente H ou $-COCH_3$ et, si R représente $-COCH_3$, le composé est un isomère optique (+),

qui consiste à

(a) faire réagir le 1-[2-(N-éthoxycarbonyl-N-méthylamino)éthyl]-1-méthyl-7-méthoxy-1,2,3,4-tétrahydronaphtalène représenté par la formule (III) suivante

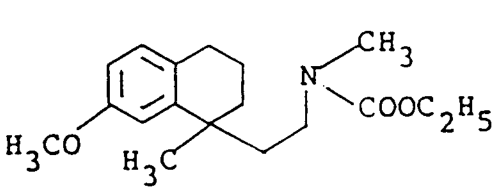 ..... (III)

avec un hydroxyde alcalin dans un solvant réactionnel pour former le (±)-1-(2-méthylaminoéthyl)-1-méthyl-7-méthoxy-1,2,3,4-tétrahydronaphtalène représenté par la formule (II) suivante

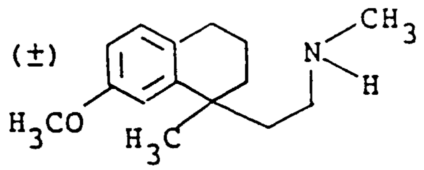

..... (II)

ou (b) dédoubler optiquement le (±)-1-(2-méthylaminoéthyl)-1-méthyl-7-méthoxy-1,2,3,4-tétrahydronaphtalène représenté par la formule (II) suivante

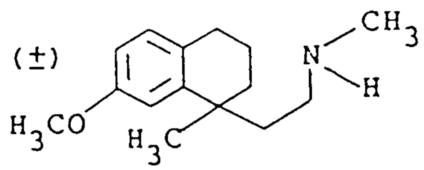

..... (II)

avec de l'acide tartrique optiquement actif dans un solvant de dédoublement pour former le (+)-1-(2-méthylaminoéthyl)-1-méthyl-7-méthoxy-1,2,3,4-tétrahydronaphtalène représenté par la formule (I) suivante

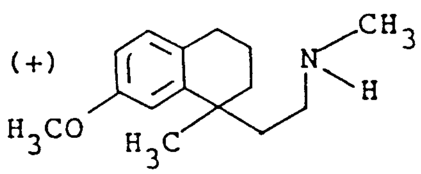

..... (I)

ou (c) mettre en contact le (+)-1-(2-méthylaminoéthyl)-1-méthyl-7-méthoxy-1,2,3,4-tétrahydronaphtalène représenté par la formule (I) suivante

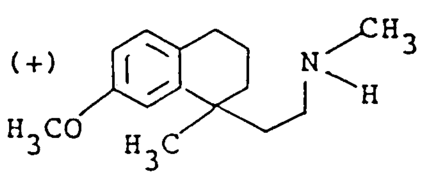

..... (I)

avec un agent acétylant dans un solvant réactionnel pour former un composé de formule (IV) suivante

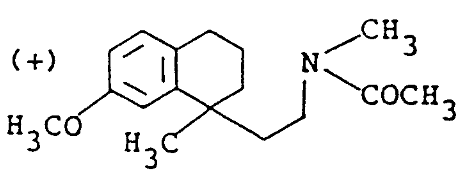

..... (IV).

**Revendications pour l'Etat contractant suivant : ES**

1. Procédé de production de (±)- ou (+)-1-[2-(amino N-substitué)éthyl]-1-méthyl-1,2,3,4-tétrahydronaphtalène représenté par la formule (A) suivante

(±) ou (+) 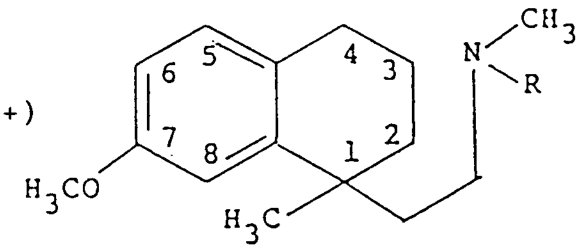 ... (A)

dans laquelle R représente H ou $-COCH_3$ et, si R représente $-COCH_3$, le composé est un Isomère optique (+),
qui consiste à

(a) faire réagir le 1-[2-(N-éthoxycarbonyl-N-méthylamino)éthyl]-1-méthyl-7-méthoxy-1,2,3,4-tétrahydronaphtalène représenté par la formule (III) suivante

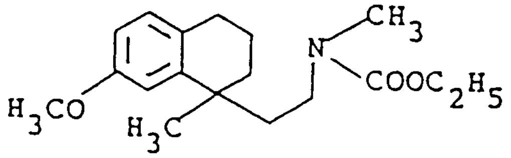 ..... (III)

avec un hydroxyde alcalin dans un solvant réactionnel pour former le (±)-1-(2-méthylaminoéthyl)-1-méthyl-7-méthoxy-1,2,3,4-tétrahydronaphtalène représenté par la formule (II) suivante

(±) 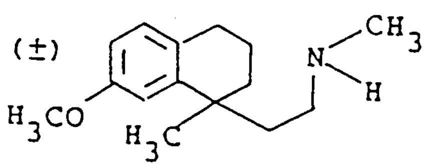 ..... (II)

ou (b) dédoubler optiquement le (±)-1-(2-méthylaminoéthyl)-1-méthyl-7-méthoxy-1,2,3,4-tétrahydronaphtalène représenté par la formule (II) suivante

(±) 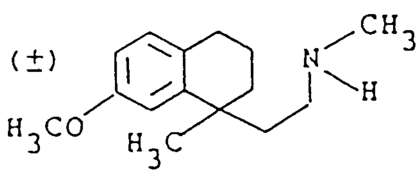 ..... (II)

avec de l'acide tartrique optiquement actif dans un solvant de dédoublement pour former le (+)-1-(2-méthylaminoéthyl)-1-méthyl-7-méthoxy-1,2,3,4-tétrahydronaphtalène représenté par la formule (I) suivante

(+) 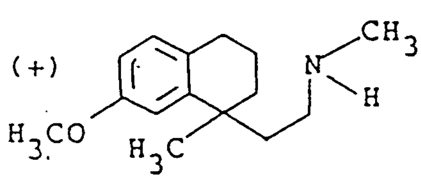 ..... (I)

ou (c) mettre en contact le (+)-1-(2-méthylaminoéthyl)-1-méthyl-7-méthoxy-1,2,3,4-tétrahydronaphtalène représenté par la formule (I) suivante

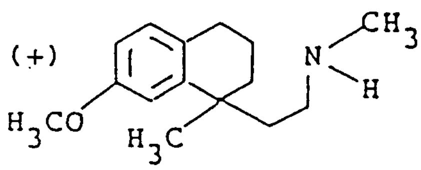

..... (I)

avec un agent acétylant dans un solvant réactionnel pour former un composé de formule (IV) suivante

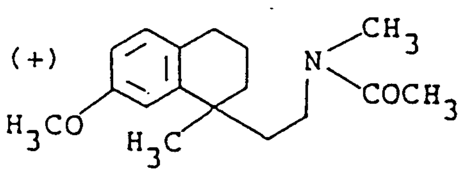

..... (IV).

2. Procédé selon la revendication 1, pour la production du (±)-1-(2-méthylaminoéthyl)-1-méthyl-7-méthoxy-1,2,3,4-tétrahydronaphtalène représenté par la formule (II) suivante :

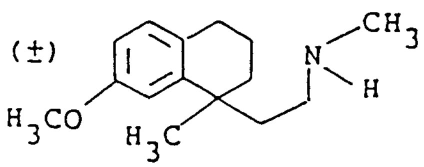

..... (II)

3. Procédé selon la revendication 1, pour la production du (+)-1-(2-méthylaminoéthyl)-1-méthyl-7-méthoxy-1,2,3,4-tétrahydronaphtalène représenté par la formule (I) suivante :

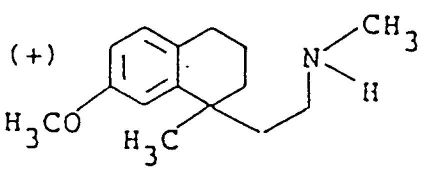

..... (I)

4. Procédé selon la revendication 1, pour la production du (+)-1-[2-(N-acétyl-N-méthylamino)éthyl]-1-méthyl-7-méthoxy-1,2,3,4-tétrahydronaphtalène représenté par la formule (IV) suivante :

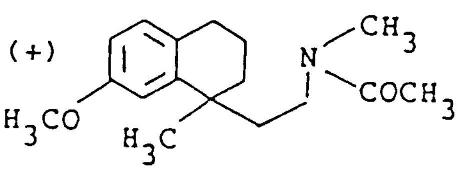

..... (IV)